(19)

**Europäisches Patentamt**
**European Patent Office**
**Office européen des brevets**

(11) **EP 4 760 331 A1**

(12) **EUROPEAN PATENT APPLICATION**

(43) Date of publication:
**17.06.2026 Bulletin 2026/25**

(21) Application number: **24219431.4**

(22) Date of filing: **12.12.2024**

(51) International Patent Classification (IPC):
**G01R 33/565** $^{(2006.01)}$     **G01R 33/56** $^{(2006.01)}$
**A61B 5/055** $^{(2006.01)}$     **G01R 33/567** $^{(2006.01)}$

(52) Cooperative Patent Classification (CPC):
**G01R 33/56509; A61B 5/7267; G01R 33/5608;**
**G01R 33/5676**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.**
**5656 AE Eindhoven (NL)**

(72) Inventors:
• **SOMMER, Karsten**
**Eindhoven (NL)**
• **MEINEKE, Jan Jakob**
**Eindhoven (NL)**
• **KEUPP, Jan**
**5656AG Eindhoven (NL)**

(74) Representative: **Philips Intellectual Property &
Standards**
**High Tech Campus 34**
**5656 AE Eindhoven (NL)**

(54) **NAVIGATOR INFORMED RETROSPECTIVE MAGNETIC RESONANCE IMAGING MOTION
CORRECTION**

(57)     Disclosed herein is a method of magnetic resonance imaging. The method comprises receiving (200) groups of k-space data descriptive. The groups of k-space data comprise group specific imaging k-space data and group specific navigator k-space data. The method further comprises determining (202) group specific subject positions using the group specific navigator k-space data of the groups of k-space data. The method further comprises initializing (204) group specific motion parameters of a joint optimization using the group specific subject positions. The method further comprises reconstructing (206) a motion corrected magnetic resonance image by performing the joint optimization on the group specific imaging k-space data of the groups of k-space data. The joint optimization is configured to simultaneously reconstruct the motion corrected magnetic resonance image while determining the group specific motion parameters. The method further comprises providing (208) the motion corrected magnetic resonance image descriptive of the field of view.

```
                                                                    400
┌─────────────────────────────────────────────────────────────┐
│ control a magnetic resonance imaging system with pulse sequence│
│ commands to acquire the groups of k-space data                │
└─────────────────────────────────────────────────────────────┘
                                                                    200
┌─────────────────────────────────────────────────────────────┐
│ receive groups of k-space data descriptive of a common field of view│
└─────────────────────────────────────────────────────────────┘
                                                                    202
┌─────────────────────────────────────────────────────────────┐
│ determine group specific subject positions using the group specific│
│ navigator k-space data of the groups of k-space data          │
└─────────────────────────────────────────────────────────────┘
                                                                    204
┌─────────────────────────────────────────────────────────────┐
│ initialize group specific motion parameters of a joint optimization│
│ using the group specific subject positions                    │
└─────────────────────────────────────────────────────────────┘
                                                                    402
┌─────────────────────────────────────────────────────────────┐
│ adjust one or more adjustable hyperparameters using the group  │
│ specific navigator data                                        │
└─────────────────────────────────────────────────────────────┘
                                                                    404
┌─────────────────────────────────────────────────────────────┐
│ identifying one or more motion corrupted groups of k-space data by│
│ comparing the group specific subject positions of the groups of│
│ k-space data to a predetermined criterion                     │
└─────────────────────────────────────────────────────────────┘
                                                                    406
┌─────────────────────────────────────────────────────────────┐
│ excluding the one or more motion corrupted groups of k-space data│
│ from the reconstruction of the motion corrected magnetic resonance image│
└─────────────────────────────────────────────────────────────┘
                                                                    408
┌─────────────────────────────────────────────────────────────┐
│ identifying one or more similar groups of k-space data by comparing the│
│ group specific subject positions of the groups of k-space data to identify│
│ similar subject positions within a predetermined threshold    │
└─────────────────────────────────────────────────────────────┘
                                                                    410
┌─────────────────────────────────────────────────────────────┐
│ combining the one or more groups of similar k-space data into combined│
│ groups of k-space data before applying joint optimization to   │
│ the groups of k-space data                                     │
└─────────────────────────────────────────────────────────────┘
                                                                    206
┌─────────────────────────────────────────────────────────────┐
│ reconstruct a motion corrected magnetic resonance image by     │
│ performing the joint optimization to the groups of k-space data│
└─────────────────────────────────────────────────────────────┘
                                                                    208
┌─────────────────────────────────────────────────────────────┐
│ provide the motion corrected magnetic resonance               │
│ image descriptive of the field of view                        │
└─────────────────────────────────────────────────────────────┘
```

Fig. 4

EP 4 760 331 A1

**Description**

TECHNICAL FIELD

The invention relates to magnetic resonance imaging, in particular to the correction of motion artifacts in magnetic resonance imaging.

BACKGROUND

A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field. Various quantities or properties of the subject can be measured spatially and imaged using MRI. A difficulty with performing magnetic resonance imaging is that it takes time to acquire the k-space data and often the subject will move causing blurring and motion artifacts in the resulting magnetic resonance image.

US2021/0364587 A1 discloses method of medical imaging including receiving k-space data that is divided into multiple k-space data groups, selecting one of the multiple k-space data groups as a reference k-space data group, and calculating spatial transform data for each of the multiple k-space data groups by inputting the multiple k-space data groups and the reference k-space data group into a transformation estimation module. The spatial transformation estimation module is configured for outputting spatial transform data descriptive of a spatial transform between a reference k-space data group and multiple k-space data groups in response to receiving the reference k-space data group and the multiple k-space data groups as input. The method further comprises reconstructing a corrected magnetic resonance image according to the magnetic resonance imaging protocol using the multiple k-space data groups and the spatial transform data for each of the multiple k-space data groups.

SUMMARY

The invention provides for a method of magnetic resonance imaging, a computer program product, and a medical system in the independent claims. Embodiments are given in the dependent claims.

In one aspect a method of magnetic resonance imaging is disclosed. The method comprises receiving groups of k-space data descriptive of a common field of view. The groups of k-space data comprise group specific imaging k-space data and group specific navigator k-space data. The method further comprises determining or ascertaining group specific subject positions using the group specific navigator k-space data of the groups of k-space data. The method further comprises initializing group specific motion parameters of a joint optimization using the group specific subject positions. The method further comprises reconstructing a motion corrected magnetic resonance image by performing the joint optimization on the group specific imaging k-space data. The joint optimization is configured to simultaneously reconstruct the motion corrected magnetic resonance image while determining the group specific motion parameters. The method further comprises providing the motion corrected magnetic resonance image descriptive of the field of view.

In an embodiment of the method, the joint optimization comprises one or more adjustable hyperparameters, which hyperparameters are adjusted using the group specific navigator data. Further embodiments are detailed throughout the description of the invention and the claims.

In another aspect, a computer program product is disclosed. The computer program product comprises a computer-readable storage medium having machine-executable instructions embodied therewith. The machine-executable instructions are configured to implement an example of the method.

In another aspect, a medical system is disclosed. The medical system comprises a computational system configured for controlling the medical system to execute an example of the method.

In another alternative, the medical system comprises a memory storing machine-executable instructions. The execution of the machine-executable instructions causes the computational system to receive groups of k-space data. The groups of k-space data comprise group specific imaging k-space data and group specific navigator k-space data. Execution of the machine-executable instructions further causes the computational system to determine a group specific subject positions using the group specific navigator k-space data of the groups of k-space data. Execution of the machine-executable instructions further causes the computational system to initialize group specific motion parameters of a joint optimization using the group specific subject positions. Execution of the machine-executable instructions further causes the computational system to reconstruct a motion corrected magnetic resonance image by performing the joint optimization on the group specific imaging k-space data. The joint optimization is configured to simultaneously reconstruct the motion corrected magnetic resonance image while determining the group specific motion parameters. Execution of the machine-executable instructions further causes the computational system to provide the motion corrected magnetic resonance image.

To be noted, that the terms "joint optimization" and "hyperparameters" are well-known in the technical field of the invention, for which evidence is the textbook by Andre van der Kouwe, Jalal B. Andre, "Motion Correction in MR: Correction of Position, Motion, and Dynamic Change" Academic Press, Paperback ISBN: 9780128244609, eBook ISBN: 9780128244784, between others particularly pages 263, 103 respectively. "Joint optimization" refers to an optimization with two targets, such as image reconstruction and coil sensitivity estimation. In the case of motion correction, it typically refers to estimating the true image and the motion trajectory at the same time, as in the invention. The term "hyperparameters", originates from the Machine Learning field, where it refers to parameters that are not part of the neural network. It is however, also used in other fields, where it typically refers to parameters that have to be set manually, e.g., that are not part of any optimization.

BRIEF DESCRIPTION OF THE DRAWINGS

In the following preferred embodiments of the invention will be described, by way of example only, and with reference to the drawings in which:

Fig. 1 illustrates an example of a medical system.
Fig. 2 shows a flow chart which illustrates a method according to the invention.
Fig. 3 illustrates a further example of a medical system.
Fig. 4 shows a flow chart which illustrates a further method according to the invention.
Fig. 5 shows a cloud-based implementation of a medical system.
Fig. 6 shows a motion corrected magnetic resonance image reconstructed using a conventional algorithm.
Fig. 7 shows a motion corrected magnetic resonance image reconstructed according to an example.

DESCRIPTION OF EMBODIMENTS

For the purposes of promoting and understanding the principles of the present disclosure, reference will now be made to the embodiments illustrated in the drawings, and specific language will be used to describe the same. It is nevertheless understood that no limitation to the scope of the disclosure is intended. Any alteration and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately. Features described in relation to a system, may be implemented in a computer implemented method and/or in a computer program product, in a corresponding manner.

Like numbered elements in these figures are either equivalent elements or perform the same function. Elements which have been discussed previously will not necessarily be discussed in later figures if the function is equivalent.

In one example, there is a method of magnetic resonance imaging. The method comprises receiving groups of k-space data descriptive of a common field of view. The groups of k-space data comprise group specific imaging k-space data and group specific navigator k-space data. The groups of k-space data may for example be k-space data that is acquired by a particular pulse sequence or group of pulse sequences that are acquired sequentially. The imaging k-space data is k-space data which is used to reconstruct the resulting motion corrected magnetic resonance imaging. The group specific navigator k-space data is navigator k-space data belonging to a particular group and providing motion data descriptive of a motion state or position of the subject. The method further comprises determining group specific subject positions using the group specific navigator k-space data of the groups of k-space data. In this step, the navigator k-space data belonging to the individual groups may be used to determine a subject position of that particular group.

The method further comprises initializing the group specific motion parameters of a joint optimization using the group specific subject positions. In a joint optimization a minimization problem is set up to reconstruct a motion corrected magnetic resonance image. Part of a minimization process is to optimize image quality while varying the motion parameters of individual groups or shots of k-space data. An effect of initializing the group specific motion parameters is to use the positions determined from the navigators to provide starting positions of the individual groups of k-space data which may be close to their optimal or true positions. This may have the effect of speeding up the solution of the joint optimization as well as ensuring, in some instances, that the position is for example not a local minimum of the optimization problem. Overall, this may have the effect of accelerating and improving image quality for magnetic resonance imaging reconstructions.

The method further comprises reconstructing a motion corrected magnetic resonance image by performing the joint optimization to or on the group specific imaging k-space data of the groups of k-space data. The joint optimization is configured to simultaneously reconstruct the motion corrected magnetic resonance image while determining the group specific motion parameters. The method further comprises providing the corrected magnetic resonance image descriptive of the field of view. Providing the motion corrected magnetic resonance image may for example entail storing it within a database or storage medium or for example provide a rendering on a display for a technologist or radiologist to examine.

As was mentioned above, one of the advantages of this method is that it may provide for improved image quality because the joint optimization is guided towards a global minimum or the correct minimum and may also be accelerated due to the fact that the joint optimization does not need to search as long or as detailed to find the minimum.

An example of a joint optimization which may be used to reconstruct a motion corrected magnetic resonance image is the following minimization problem:

$$\hat{x} = \underset{x,\,\theta}{arg\,min}\|AFCT(\theta)x - y\|_2^2 \qquad (1)$$

where $x$ is the image to be estimated, $y$ is the acquired k-space data, $\theta$ is a set of motion parameters, $T$ is the motion transform matrix, $C$ are the coil sensitivities, $F$ is the Fourier transform and $A$ is the sampling matrix. Eq. (1) can be solved in an alternating fashion, i.e. by solving the subproblem of estimating $x$ while keeping $\theta$ fixed (image estimation), and vice versa (motion estimation).

The numerical solution of Eq. (1) is mathematically challenging because in addition to the unknown image $x$, the motion trajectory of the patient during the scan may be estimated as well. Without any prior information on the patient motion, the scan time is typically divided into n_segm segments of equidistant length. All data acquired in the time frame of such a segment is assumed to belong to a motion state described by one set of motion parameters. The number of these employed segments (n_segm) can be freely chosen but represents a trade-off between reconstruction time and achievable image quality: a high number of segments is typically needed to accurately resolve the patient's motion trajectory, but reconstruction time increases linearly with n_segm. Even with a relatively high value of n segm, abrupt motion events often cannot be resolved accurately, leading to residual motion artifacts in the motion-compensated image.

A further parameter that is difficult to adequately choose for the motion compensation algorithm is the step size $\alpha$ of the motion estimation algorithm: for large motion events, a large value of $\alpha$ is ideal to quickly converge to the quick solution; for small or no motion events, a smaller value of $\alpha$ is ideal to avoid large oscillations around the correct value.

In another example, the group specific navigator k-space data has the same k-space data sampling pattern for all groups of k-space data. In this example, the group specific navigator k-space data has the same sampling locations used for the various groups of k-space data. An advantage of this is that the navigator does not vary from group to group. In some instances, this may provide for a more accurate determination of the group specific motion parameters. It may also provide for an improved means of comparing the different groups of k-space data. For example, if the k-space data is later binned together to reduce the number of groups and thereby also accelerate the solution of the joint optimization, the raw k-space data itself may be compared if the sampling locations are the same. It provides for maybe a direct analysis of the k-space data whereas in other techniques where the location of the k-space data is varied.

In another example, the determination or ascertaining of the group specific subject positions using the group specific navigator k-space data excludes self-navigation. This example may be beneficial because the navigator k-space data may be chosen based on its ability to determine the subject position. This may for example be useful in ensuring that the group specific motion parameters are more accurately determined as opposed to choosing the navigator k-space data on its ability to augment the group specific imaging k-space data.

In another example, the group specific k-space data is separate from the group specific navigator k-space data. With this example, the group specific navigator k-space data may also be chosen or designed such that it may provide for better group specific motion parameters as opposed to augmenting the reconstruction of the motion corrected magnetic resonance image with additional k-space data. This may, for example, lead to better image reconstruction overall.

In another example, the joint optimization comprises one or more adjustable hyperparameters. The method further comprises adjusting the one or more adjustable hyperparameters using the group specific navigator data. Often joint optimizations will be solved using variants of gradient descent solvers. One hyperparameter which would be adjustable for a gradient-based solver would be the step size. By examining the group of specific motion parameters, one could for example have an idea of the amount of motion between the different group specific subject positions. Using this information, one then could map this onto various values of the adjustable hyperparameters. An advantage of using the adjustable hyperparameters is that the joint optimization may for example converge to a solution more rapidly as well as choosing a bare minimum for the optimization problem. This could, again, result in a reduced amount of computational burden as well as providing for a higher quality reconstruction of the motion corrected magnetic resonance image.

In another example, the joint optimization is solved using a gradient-based solver. The one or more hyperparameters comprise a step size. As was mentioned above, choosing the proper step size may have the benefit of improving not only the time to convergence but also may provide for a better quality magnetic resonance image.

For example, an estimate of the 3D rotation of the patient anatomy can be calculated using only the magnitude of the orbital navigator data. After this, 3D translation parameters are estimated using the navigator phase data. Using the resulting 3D motion trajectory, an estimate of the motion magnitude is computed, e.g., the maximum of all 6 motion parameters for each segment. The step size of the motion estimation algorithm is then adjusted for each segment based on the expected motion magnitude (large expected motion may result in a large step size, and vice versa). This way, the algorithm may converge quickly to the correct solution for segments with small and large motion events, which benefits overall reconstruction time

In another example, the joint optimization is solved using a gradient-based solver and a moving average of past gradients is added to an update vector. The one or more hyperparameters comprises the step size and/or a scaling factor that determines the effect of the average of the past gradients on the update factor. A knowledge of the change in the group specific positions between adjacent groups can for example be used to select the step size as well as being mapped onto a scaling factor. Again, this may provide for improved image quality as well as more computational efficiency.

In another example, the joint optimization is solved using a Root Mean Squared Propagation (RMSprop) solver or an Adam solver (a stochastic gradient descent method that is based on adaptive estimation of first-order and second-order moments). The joint optimization is configured to use a running average of squared gradients to scale the step size. The one more hyperparameters may comprise the step size and/or a discount factor configured to determine a relative impact of current and previous squared gradients. Likewise, this may provide for improved image quality as well as a decreased amount of time to converge to a solution.

In another example, the method further comprises identifying one or more motion corrected groups of k-space data by comparing the group specific subject positions of the groups of k-space data to a predetermined criterion. For example, one of the groups of k-space data could be considered to be a reference position. Often, the first acquired k-space data may be selected, however a different group may also be selected. The position data from the other groups may then be compared to this reference group and if a particular group varies by more than a particular amount or by a particular metric, then it can be identified as being motion corrupted.

The method further comprises excluding the one or more motion corrupted groups of k-space data from the reconstruction of the motion corrected magnetic resonance image. For example, if there is some repetitious motion or periodic motion of the subject, excluding one or more of the motion corrupted groups may have the effect of providing for an improved motion corrected magnetic resonance image.

In another example, the method further comprises determining an expected reconstruction time and/or estimated image quality classification using the group specific subject positions. The method further comprises displaying the expected reconstruction time and/or the estimated image quality on a user interface. The method further comprises receiving a continue selection or reacquire selection from the user interface. The method further comprises triggering a magnetic resonance imaging system to reacquire the groups of k-space data if the reacquire selection is received. The method further comprises enabling the reconstruction of the motion corrected magnetic resonance image if the continue selection is received. This example may be beneficial because it may provide for a means for an operator to decide to continue with the reconstruction or to reacquire it if the motion of the subject is excessive.

In another example, the method further comprises modifying one or more image reconstruction parameters by comparing the group specific subject positions of the group of k-space data to a predetermined mapping. The one or more image reconstruction parameters comprise any one of the following: a maximum number of iterations of the joint optimization, a choice of a type of algorithm used for solving the joint optimization, a number of coil elements used during the reconstruction, a convergence criterion for the joint optimization, a regularization parameter, a reduction scheme for the step size, a weighting factor, and combinations thereof. All of these various parameters may be effectively adjusted using the group specific subject positions to ensure that the joint optimization functions better and may also converge more rapidly.

In another example, the method further comprises identifying one or more similar groups of k-space data by comparing the group specific subject positions of the groups of k-space data to identify similar subject positions within a predetermined threshold. The method further comprises combining the one or more groups of k-space data into combined groups of k-space data before applying the joint optimization to the groups of k-space data. In this example, if groups of k-space data have a similar enough motion state, then they may be effectively combined. This has the effect of reducing the number of groups in the optimization. This may have a huge effect on accelerating the speed of the solution of the joint optimization.

In another example, the group specific subject positions are received in response to inputting the group specific navigator k-space data into a trained machine learning module. For example, the trained machine learning module may be a convolutional neural network. The convolutional neural network could be trained to output the group specific positions in response to receiving the group specific navigator k-space data. This for example may be trained by having labeled pairs of navigator k-space data which is input and then compared against actual positions as ground truth data. This data may for example be used to train the trained machine learning module using a deep learning procedure.

In another example, the group specific navigator k-space data is any one of the following: one-dimensional navigator k-space data, orbital navigator k-space data, and spherical navigator k-space data. All of these various types of navigators may for example be useful in determining the subject position. The orbital navigator k-space data and the spherical navigator k-space data may have the benefit of providing movement in one or more dimension.

In another example, the method further comprises acquiring the groups of k-space data from a field of view by controlling a magnetic resonance imaging system with pulse sequence commands. The pulse sequence commands are configured to separately acquire the group specific imaging k-space data and the group specific navigator k-space data.

In another example, the method is implemented by providing a computer program product that comprises a computer-readable storage medium having machine-executable instructions embodied therewith. All of the various methods described above may be computer-implemented methods and as such, may be implemented as computer program product.

In another example, there is a medical system which may comprise a memory storing machine-executable instructions. The machine-executable instructions are configured to implement an example of a method described above. The medical system further comprises a computational system configured for controlling the medical system. The execution of the machine-executable instructions causes the computational system to perform an example of a method as described above.

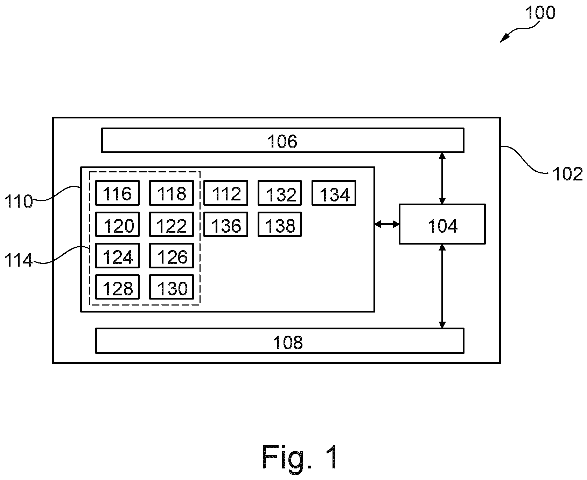

Fig. 1 illustrates an example of a medical system 100. The medical system 100 is shown as comprising a computer 102. The computer 102 is intended to represent one or more computers or computer systems located at one or more locations. The computer 102 is shown as comprising a computational system 104. Likewise, the computational system 104 may represent one or more computational systems, processors, or cores located at one or more locations. For example, the computer 102 and computational system 104 could comprise multiple computer systems at multiple locations.

The computational system 104 is shown as being in communication with an optional hardware interface 106 or network interface. The hardware interface 106 may for example be used to control or exchange data with other components of the medical system 100 if they are present. The computational system 104 is further shown as being in communication with an optional user interface 108. The user interface 108 may for example be used by an operator to control the operational and function of the medical system 100.

The computational system 104 is further shown as being in communication with a memory 110. The memory 110 is intended to represent various types of memory which are accessible to the computational system 104. In some examples the memory 110 is a non-transitory storage medium. The memory 110 in some embodiments may be based on or may rely on cloud-based data stored in logical pools across disparate, commodity storage servers located on premises or in a data center managed by a third-party cloud provider. Accordingly, the memory as schematically depicted in Fig. 1 is just for illustration purposes. Therefore, any or all components comprised in memory 110 may be cloud-based.

The memory 110 is shown as storing machine-executable instructions 112. The machine-executable instructions 112 may enable the computational system 104 to perform various tasks such as controlling other components of the medical system 100, performing image processing and numerical tasks, and also implementing such things as a method of magnetic resonance imaging or image processing for such a method.

The memory is further shown as containing groups of k-space data 114. Each group is shown as comprising separate imaging and navigator k-space data. There is a first group of imaging k-space data 116 that corresponds to a first group of navigator k-space data 118. There is a second group of imaging k-space data 120 that corresponds to a second group of navigator k-space data 122. There is a third group of imaging k-space data 124 that corresponds to a third group of navigator k-space data 126. There is a fourth group of imaging k-space data 128 that corresponds to a fourth group of navigator k-space data 130. The exact number of groups is essentially arbitrary and dependent upon the particular magnetic resonance imaging protocol and acquisitions. In this example, it is clear that the groups of imaging k-space data 116, 120, 124, and 128 are separate from the navigator k-space data 118, 122, 126, and 130.

The memory 110 is further shown as storing group specific subject positions 132 that were determined from the groups of navigator k-space data 118, 122, 126, and 130. The memory 110 is further shown as containing group specific subject positions 132 that were determined from the navigator k-space data 118, 122, 126, and 130. In this particular example, the group specific subject positions 132 is used to initialize a group specific motion parameters 134 used in a joint optimization 136. The memory 110 is further shown as storing a motion corrected magnetic resonance image 138 that was reconstructed using the joint optimization module 136 to operate on the groups of imaging k-space data 116, 120, 124, and 128. The group specific subject positions 132 were used to initialize the group specific motion parameters 134.

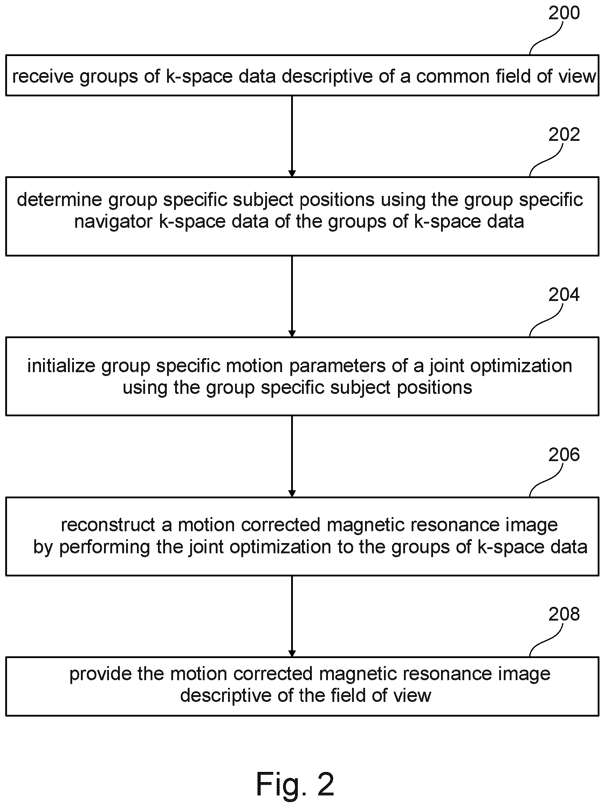

Fig. 2 shows a flowchart which illustrates a method of operating a medical system, for example the one schematically illustrated in Fig. 1. In step 200, the groups of k-space data 114 are received and are descriptive of a common field of view of a subject. In step 202, the group specific subject positions 132 are determined from the group specific navigator k-space data 118, 122, 126, 130. In step 204, the group specific motion parameters 134 of the joint optimization 136 are initialized using the group specific subject positions 132. In step 206, the motion corrected magnetic resonance image 138 is reconstructed by performing the joint optimization on the groups of k-space data 116, 120, 124, 128.

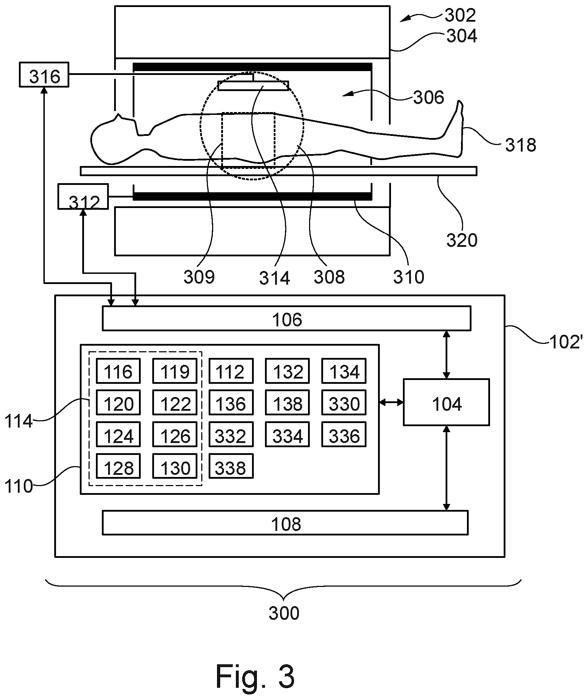

Fig. 3 illustrates a further example of a medical system 300. The medical system 300 is similar to the medical system 100 depicted in Fig. 1 except that it additionally comprises a magnetic resonance imaging system 302. The medical system 300 of Fig. 3 also comprises a computer 102', which is similar to the computer 102 depicted in Fig. 1. The computer 102' is used to additionally control the magnetic resonance imaging system 302. The computer 102 in Fig. 1 may also be modified to perform the imaging processing and image reconstruction functionality of computer 102'.

The magnetic resonance imaging system 302 comprises a magnet 304. The magnet 304 is a superconducting cylindrical type of magnet with a bore 306 through it. It is also possible to use both a split cylindrical magnet and a so-called open magnet. A split cylindrical magnet is similar to a standard cylindrical magnet, except that the cryostat has been split into two sections to allow access to the iso-plane of the magnet. Such magnets may for instance be used in conjunction with charged particle beam therapy. An open magnet has two magnet sections, one above the other with a space in-between that is large enough to receive a subject. The arrangement of the two magnet sections is similar to that of a Helmholtz coil. Open magnets are popular because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The k-space data are acquired for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309.

Within the bore 306 of the magnet there is also a set of magnetic field gradient coils 310 which is used for the acquisition of k-space data to spatially encode magnetic spins within the imaging zone 308 of the magnet 304. The magnetic field gradient coils 310 are connected to a magnetic field gradient coil power supply 312. The magnetic field gradient coils 310 are intended to be representative. Typically, magnetic field gradient coils 310 contain three separate sets of coils for spatial encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio frequency coil 314 is connected to a radio frequency transceiver 316. The radio frequency coil 314 and radio frequency transceiver 316 may be replaced by separate transmit and receive coils and a separate transmitter and receiver. It is understood that the radio frequency coil 314 and the radio frequency transceiver 316 are representative. The radio frequency coil 314 is intended to also represent a dedicated transmit antenna and a dedicated receive antenna. Likewise, the transceiver 316 may also represent a separate transmitter and receiver. The radio frequency coil 314 may also have multiple receive/transmit elements and the radio frequency transceiver 316 may have multiple receive/transmit channels. The transceiver 316 and the gradient coil power supply 312 are shown as being connected to the hardware interface 106 of the computer system 102'.

The memory 110 can be comprised in the computer system 102' or can be cloud-based, similar to Fig. 1. The memory 110 is further shown as storing pulse sequence commands 330. The pulse sequence commands 330 control the magnetic resonance imaging system 302 according to a magnetic resonance imaging protocol to acquire the groups of k-space data 114. The memory 110 is further shown as containing optional features 332, 334, 336, and 338. The memory 110 is shown as storing the adjustable hyperparameters 332. These are parameters which may be used to adjust the behavior of the joint optimization 136. As was discussed above, these may for example be a step size used in a gradient descent or other parameters. The memory 110 is also shown as containing identified motion corrupted groups of k-space data 334 that were identified using the group specific subject positions 132. During the construction of the motion corrected magnetic resonance image 138, the identified motion corrupted groups of k-space data 334 may be excluded.

The memory 110 is further shown as containing identified similar groups of k-space data 336 that were identified by comparing the group specific subject positions 132. This may for example identify groups of k-space data which have a similar position and therefore may be grouped together or combined during the solution of the joint optimization 136. Feature 338 in the memory 110 represents one or more groups of k-space data that have been combined together.

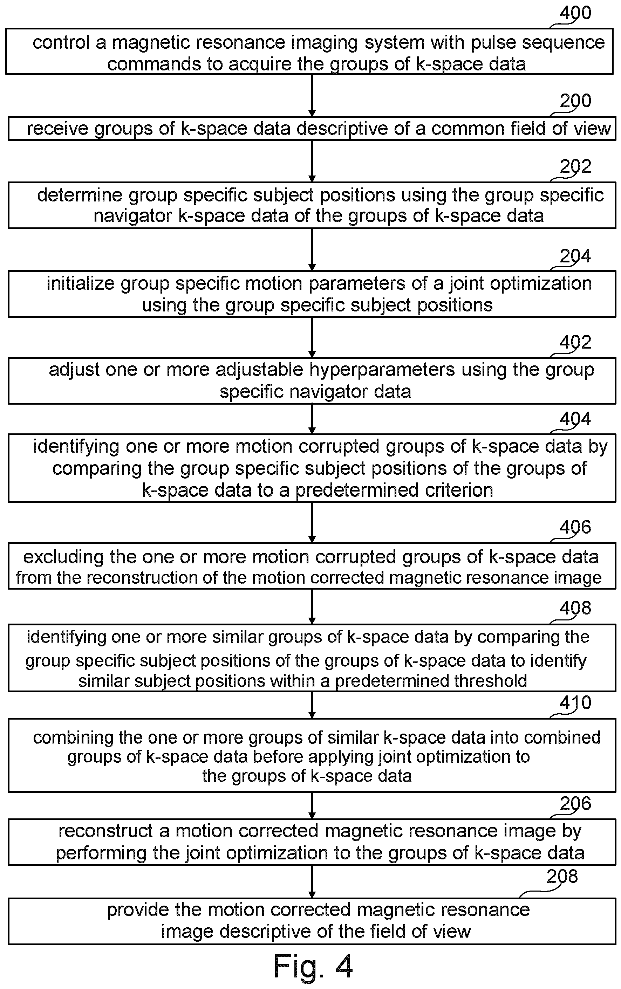

Fig. 4 shows a flowchart which illustrates a method of operating the medical system 300 of Fig. 3. In step 400, the magnetic resonance imaging system 302 is controlled with the pulse sequence commands 330 to acquire the groups of k-space data 114. Steps 200, 202, and 204 are performed as was illustrated in Fig. 2. In step 402, the one or more adjustable hyperparameters 332 are adjusted using the group specific navigator data 118, 122, 126, 130. In some examples, the group specific navigator data 118, 122, 126, 130 is used directly. In other cases the group specific subject positions 132 are used. In step 404, the one or more motion corrupted groups of k-space data 334 are identified. This is performed by comparing the group specific subject positions 132 to a predetermined criterion.

This may for example be one of the group specific subject positions 132 which is selected as a reference position. In step 406, the one or more motion corrupted groups of k-space data 334 are excluded from the reconstruction of the motion corrected magnetic resonance image 138. In step 408, the one or more similar groups of k-space data 336 are identified by comparing the group specific subject positions 134 to identify similar subject positions within a predetermined threshold. In step 410, the one or more groups of similar k-space data 336 are combined into combined groups of k-space data 338 before applying the joint optimization 136. Steps 206 and 208 are then performed. It should be noted that step 204 forms one means of improving the joint optimization. Step 402 provides a second means of improving the joint optimization. Steps 404 and 406 provide a third means of improving the joint optimization. Steps 408 and 410 provide a fourth means of improving the joint optimization. These four different means of improving the joint optimization may be performed singularly or in different combinations. For example, in one example only step 402 is performed.

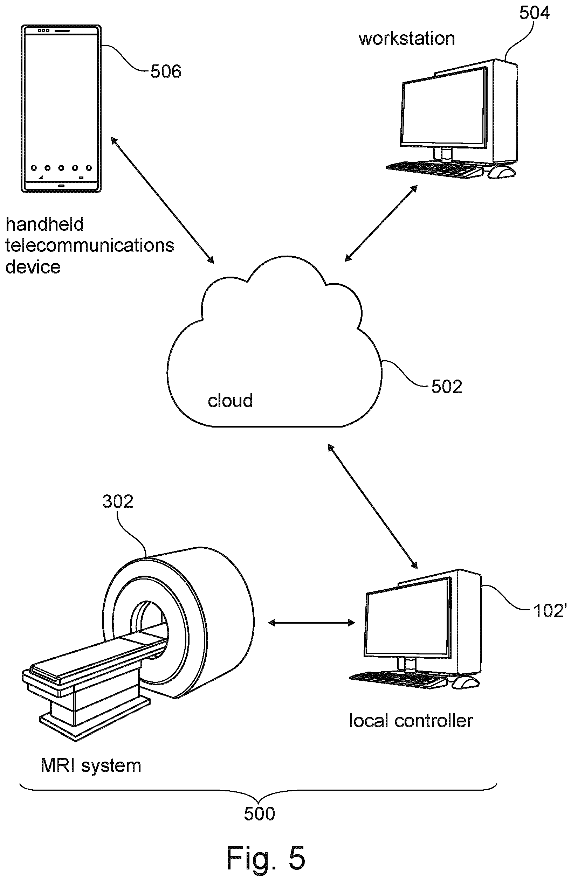

Fig. 5 illustrates a further example of a medical system 500. The medical system 500 comprises the magnetic resonance imaging system 302 and local controller 102' as well as a cloud-based computing system 502, a radiology workstation 504, a handheld telecommunications device 506, and a local controller 102. The functionality of the computer 102 in Figs. 1 and the image processing features of the computer 102' (local controller) in Fig. 3 can be distributed amongst the various devices 102', 502, 504, and 506 in Fig. 5. The cloud-based computing system 502 could be formed of networked servers and/or virtual machines available over the internet or other network.

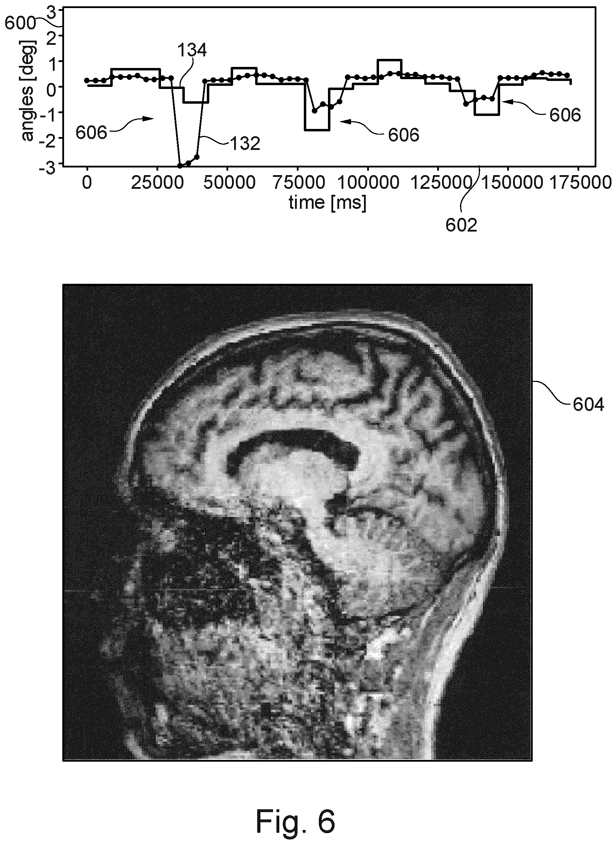

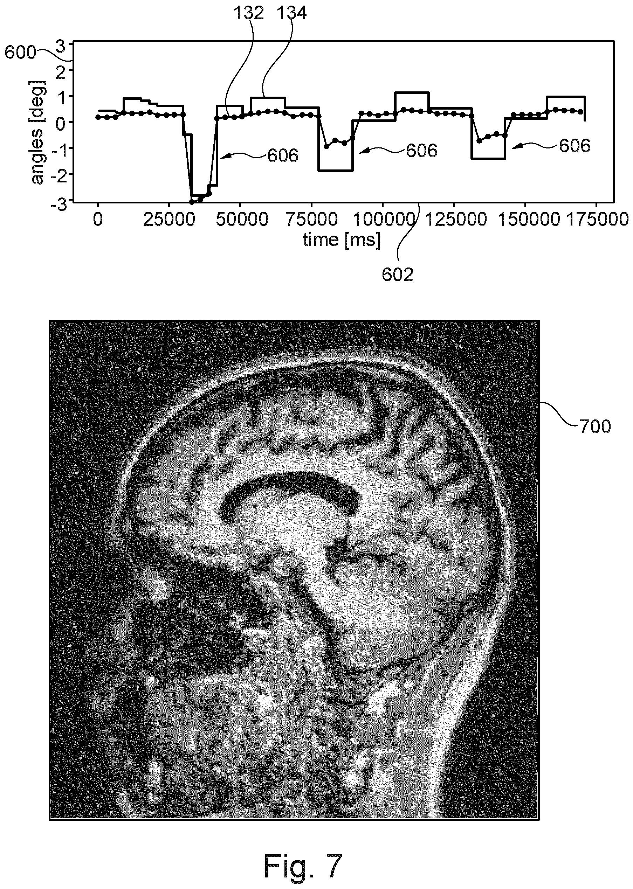

Figs. 6 and 7 illustrate the benefits of steps 408 and 410 in Fig. 4. Fig. 6 shows an example of a motion corrected image that was reconstructed using a joint optimization but did not incorporate the examples described above. It can be seen that the motion corrected image 604 is kind of grainy and several of the anatomical features are not very crisp or distinct. Above this is a chart which illustrates the position 600 as a function of time 602. In the calculations used for the image 604, there were three such optimizations for the x, y, and z positions. In this example only the x coordinate 600 is shown. The line 132 illustrates the group specific subject positions as a function of rotation about the x axis. The lines 134 represent the group specific motion parameters 134 as determined by running the joint optimization 136. It can be seen that the group specific motion parameters 134 do not match the group specific subject positions 132 where there are regions of large motion as indicated by the group specific subject positions 132. These regions are labeled 606.

Fig. 7 shows a further motion corrected image 700 that was reconstructed incorporating steps 408 and 410 as illustrated in Fig. 4. It can be seen that the motion corrected image 700 is much clearer and has finer detail than the motion corrected image 604. When comparing the specific subject positions 132 and the group specific motion parameters 134 as determined by the joint optimization 136, it can be seen that these motion parameters and values 132, 134 match much better. Although this improvement is only illustrated for steps 408 and 410, similar improvements may be expected by performing step 204 and/or step 402 and/or the combination of steps 404 and 406.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit", "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electromagnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational systems to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the C programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a Local Area Network (LAN) or a Wide Area Network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special-purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, WLAN connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, television screen, touch screen, tactile electronic display, Braille screen, Cathode Ray Tube (CRT), storage tube, bi-stable display, electronic paper, vector display, flat panel display, Vacuum Fluorescent (VD) display, Light-Emitting Diode (LED) displays, Electro-Luminescent Display (ELD), Plasma Display Panels (PDP), Liquid Crystal Display (LCD), Organic Light-Emitting Diode (OLED) displays, a projector, and head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method of magnetic resonance imaging, wherein the method comprises:

   - receiving (200) groups of k-space data descriptive of a common field of view, wherein the groups of k-space data comprise group specific imaging k-space data and group specific navigator k-space data;
   - determining (202) group specific subject positions using the group specific navigator k-space data of the groups of k-space data;
   - initializing (204) group specific motion parameters of a joint optimization using the group specific subject positions;
   - reconstructing (206) a motion corrected magnetic resonance image by performing the joint optimization on the group specific imaging k-space data of the groups of k-space data, wherein the joint optimization is configured to simultaneously reconstruct the motion corrected magnetic resonance image while determining the group specific motion parameters; and
   - providing (208) the motion corrected magnetic resonance image descriptive of the field of view.

2. The method of claim 1, wherein the group specific navigator k-space data has the same k-space sampling pattern for all groups of k-space data.

3. The method of any one of the preceding claims, wherein determining the group specific subject positions using the group specific navigator k-space excludes self-navigation.

4. The method of any one of the preceding claims, wherein the group specific k-space data is separate from the group specific navigator k-space data.

5. The method of any one of the preceding claims, wherein the joint optimization comprises one or more adjustable hyperparameters (332), wherein the method further comprises adjusting (402) the one or more adjustable hyperparameters using the group specific navigator data.

6. The method of claim 5, wherein any one of the following:

   - wherein the joint optimization is solved using a gradient based solver, wherein the one or more hyperparameters comprise a step size;
- wherein the joint optimization is solved using the gradient based solver and a moving average of past gradients is added to an update vector, wherein the one of more hyperparameters comprise the step size and/or a scaling factor that determined the effect of the average of the past gradients on the update vector; and
- wherein the joint optimization is solved using a RMSprop solver or an Adam solver, wherein the joint optimization is configured to use a running average of squared gradients to scale the step size, wherein the one or more hyperparameters comprise the step size and/or a discount factor configured to determine a relative impact of current and previous squared gradients.

7. The method of any one of the preceding claims, wherein the method further comprises:

- identifying (404) one or more motion corrupted groups of k-space data (334) by comparing the group specific subject positions of the groups of k-space data to a predetermined criterion;
- excluding (406) the one or more motion corrupted groups of k-space data from the reconstruction of the motion corrected magnetic resonance image.

8. The method of any one of the preceding claims, wherein the method further comprises:

- determining an expected reconstruction time and/or an estimated image quality classification using the group specific subject positions; and
- displaying the expected reconstruction time and/or the estimated image quality on a user interface;
- receiving a continue selection or a reacquire selection from the user interface;
- triggering a magnetic resonance imaging system to reacquire the groups of k-space data if the reacquire selection is received; and
- enabling the reconstruction of the motion corrected magnetic resonance image if the continue selection is received.

9. The method of any one of the preceding claims, wherein the method further comprises modifying one or more image reconstruction parameters by comparing the group specific subject positions of the groups of k-space data to a predetermined mapping, wherein the one or more image reconstruction parameters comprises any one of the following: a maximum number of iterations of the joint optimization, a choice of a type of algorithm used for solving the joint optimization, a number of coil elements used during the reconstruction, a convergence criterion for the joint optimization, a regularization parameter, a reduction scheme for the step size, and combinations thereof.

10. The method of any one of the preceding claims, wherein the method further comprises:

- identifying (408) one or more similar groups of k-space data (336) by comparing the group specific subject positions of the groups of k-space data to identify similar subject positions within a predetermined threshold;
- combining (410) the one or more similar groups of k-space data into a combined groups of k-space data (338) before applying the joint optimization to the groups of k-space data.

11. The method of any one of the preceding claims, wherein the group specific subject positions are received in response to inputting the group specific navigator k-space data into a trained machine learning model.

12. The method of any one of the preceding claims wherein the group specific navigator k-space data is any one of the following: one dimensional navigator k-space data, orbital navigator k-space data, and spherical navigator k-space data.

13. The method of any one of the preceding claims, wherein the method further comprises acquiring the groups of k-space data from a field of view by controlling (400) a magnetic resonance imaging system (302) with pulse sequence commands (330), wherein the pulse sequence commands are configured to separately acquire the group specific imaging k-space data and the group specific navigator k-space data.

14. A system for imaging at least a part of a body, comprising a computational system configured to perform a method according to any of the claims 1 to 13.

15. A computer program comprising machine executable instructions, wherein execution of the machine executable instructions causes a computational system to perform a method according to any of the claims 1 to 13.

Fig. 1

```
                                                                    200

receive groups of k-space data descriptive of a common field of view

                                                                    202

determine group specific subject positions using the group specific
           navigator k-space data of the groups of k-space data

                                                                    204

initialize group specific motion parameters of a joint optimization
              using the group specific subject positions

                                                                    206

reconstruct a motion corrected magnetic resonance image
by performing the joint optimization to the groups of k-space data

                                                                    208

provide the motion corrected magnetic resonance image
              descriptive of the field of view
```

Fig. 2

Fig. 3

400

control a magnetic resonance imaging system with pulse sequence commands to acquire the groups of k-space data

200

receive groups of k-space data descriptive of a common field of view

202

determine group specific subject positions using the group specific navigator k-space data of the groups of k-space data

204

initialize group specific motion parameters of a joint optimization using the group specific subject positions

402

adjust one or more adjustable hyperparameters using the group specific navigator data

404

identifying one or more motion corrupted groups of k-space data by comparing the group specific subject positions of the groups of k-space data to a predetermined criterion

406

excluding the one or more motion corrupted groups of k-space data from the reconstruction of the motion corrected magnetic resonance image

408

identifying one or more similar groups of k-space data by comparing the group specific subject positions of the groups of k-space data to identify similar subject positions within a predetermined threshold

410

combining the one or more groups of similar k-space data into combined groups of k-space data before applying joint optimization to the groups of k-space data

206

reconstruct a motion corrected magnetic resonance image by performing the joint optimization to the groups of k-space data

208

provide the motion corrected magnetic resonance image descriptive of the field of view

Fig. 4

506

handheld
telecommunications
device

504

workstation

cloud

502

302

102'

local controller

MRI system

500

Fig. 5

Fig. 6

Fig. 7

Europäisches
Patentamt

European
Patent Office

Office européen
des brevets

# EUROPEAN SEARCH REPORT

**Application Number**

EP 24 21 9431

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 468 011 A1 (KONINKLIJKE PHILIPS NV [NL]) 27 November 2024 (2024-11-27) | 1-5,7,8, 10-15 | INV.<br>G01R33/565<br>G01R33/56 |
| A | * paragraph [0005] - paragraph [0029]; figures 4,4a,5 *<br>* paragraph [0056] - paragraph [0057] *<br>* paragraph [0066] - paragraph [0077] *<br>* paragraph [0081] - paragraph [0086] *<br>* paragraph [0097] - paragraph [0098] *<br>----- | 6,9 | ADD.<br>A61B5/055<br>G01R33/567 |
| A | US 2014/126796 A1 (CHESNEAU NICOLAS [FR] ET AL) 8 May 2014 (2014-05-08)<br>* paragraph [0009] - paragraph [0022] *<br>* paragraph [0052] - paragraph [0067] *<br>* paragraph [0078] - paragraph [0086] *<br>----- | 6,9 | |

**TECHNICAL FIELDS SEARCHED (IPC)**

G01R
A61B

The present search report has been drawn up for all claims

| Place of search | Date of completion of the search | Examiner |
|---|---|---|
| Munich | 27 April 2025 | Lebar, Andrija |

EPO FORM 1503 03.82 (P4C01)

## ANNEX TO THE EUROPEAN SEARCH REPORT
## ON EUROPEAN PATENT APPLICATION NO.

EP 24 21 9431

This annex lists the patent family members relating to the patent documents cited in the above-mentioned European search report.
The members are as contained in the European Patent Office EDP file on
The European Patent Office is in no way liable for these particulars which are merely given for the purpose of information.

27-04-2025

| Patent document cited in search report | Publication date | Patent family member(s) | Publication date |
|---|---|---|---|
| EP 4468011 A1 | 27-11-2024 | NONE | |
| US 2014126796 A1 | 08-05-2014 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 20210364587 A1

**Non-patent literature cited in the description**

• **ANDRE VAN DER KOUWE** ; **JALAL B. ANDRE**. Motion Correction in MR: Correction of Position, Motion, and Dynamic Change. Academic Press